# EUROPEAN PATENT APPLICATION

(11) **EP 2 801 403 A1**
(43) Date of publication of application: **12.11.2014**
(21) Application number: 14166661.0
(22) Date of filing: 30.04.2014
(51) Int. Cl.: B01F 15/06, C12G 1/028, C12M 1/06, C12M 1/02

(54) **Primary fermentation tank with a mechanical cooling and/or degassing device**

(30) Priority: 06.05.2013 SK 50152013
(71) Applicant: Hrdlicka, Anton, 81103 Bratislava (SK)
(72) Inventor: Hrdlicka, Anton, 81103 Bratislava (SK)
(74) Representative: Cechvalova, Dagmar

(57) **Abstract**

A primary fermentation tank with a mechanical cooling and/or degassing device comprising a vessel fitted with a bottom discharge opening, the design feature of which tank is that attached to the vessel walls, housed in bearings, is a driven horizontal shaft (1) at both ends of which are blind axial holes (2) with radial outlets (3). Fixedly attached to these outlets (3) on the shaft (1) are outer tubular arms (4). In its middle part, the shaft (1) is provided with spaced radial through holes angularly offset from one another, connected to which are inner tubular arms (5), wherein the adjacent ends of the inner tubular arms (5) are continually interconnected by interconnecting tubular links (6) to form a three-dimensional tubular structure (17). Connected to the outlets of the end interconnecting tubular links (6) are the end parts of the outer tubular arms (4). The blind holes (2) at both ends of the shaft (1) are fitted with insert or sleeve couplers (7, 16) for inlet and outlet of coolant.

## Description

### Field of the invention

The present invention relates to a design of a primary fermentation tank with a mechanical cooling and/or degassing device for primary fermentation of grape must in wine making industry. This design of essentially a slow-rotating trudging mechanism performs must degassing. If a coolant is circulated through the internal channel of this three-dimensional structure, it also performs the function of a must bulk cooler. The invention falls within the field of industrial wine processing.

### State of the art

Known are grape must primary fermentation tanks in which fermentation gasses produced during the fermentation process in the form of bubbles adhere to solid particles of must (grapes, skins, pulp) which are carried up to the must surface. Gradual layering of these particles produces a hardly permeable pomace cap on the surface preventing a free escape of further produced fermentation gas. This gas gets trapped under the pomace cap, creating a buoyancy force pushing the pomace cap above the must surface. Such part of the pomace cap loses its contact with the liquid phase - the juice, preventing it from extracting dyes, bouquet and aromatic substances from the cap. In addition, high temperatures are generated in the dry part of the pomace cap causing undesirable biological processes.

That is why primary fermentation tanks are equipped with various mechanical systems designed to periodically disturb the pomace cap to enable fermentation gases to escape and thereby the pomace cap to be suspended. One known solution, for example, is a horizontal primary fermentation tank Rotatank HNN1 equipped with a motor, gear box, and low-speed stirrer consisting of two helices of different diameters and opposite gradients.

A disadvantage of such designed primary fermentation tanks is their low extraction of bouquet and aromatic substances due to reduced contact of juice with the pomace cap, higher potential for wine defects in later production stages as a result of higher fermentation temperature, development of off-flavours as a result of a dried cap being attacked by fungi and development of bitterness as a result of mechanic interference and violent disruption of the pomace cap.

Persistent problems in red wine production created a space for designing a primary fermentation tank that would ensure necessary degassing and possibly also cooling of must. The result of this effort is the primary fermentation tank with a mechanical cooling and/or degassing device according to the present invention, as described below.

### Subject matter of the invention

The above discussed disadvantages of grape processing and namely red wine production facilities are eliminated by a primary fermentation tank with a mechanical cooling and/or degassing device according to the present invention. The primary fermentation tank is usually a vertical cylindrical vessel with an open top, conical bottom and three-legged support. At the lowest point of the tank is a discharge opening fitted with a door. Horizontally oriented tanks can also be used. The essence of the invention is placing a rotary degassing device inside the primary fermentation tank. Attached to the exterior at the top of the primary fermentation tank is an electric motor that drives, via a chain transmission, the degassing device housed in bearings. An essential design feature is that the degassing device forms a three-dimensional tubular structure and comprises a shaft at both ends of which are blind holes with radial outlets and welded to these outlets at the shaft are outer tubular arms with closed ends. In the middle part of the shaft, extending transversely through it, are inner tubular arms, which are also closed at the ends, wherein adjacent inner tubular arms, which may be divided or undivided, are always angularly offset from one another and are interconnected by interconnecting tubular links. Another design feature is that the rotary shaft of the degassing device is mounted horizontally below the must surface at a distance less than half of the maximum diameter of the 3-dimensional tubular structure. With such arrangement all tubular arms and their tubular links trudge through the must while partially emerging above the level, which creates escape routes for fermentation gases allowing them to escape continuously throughout the fermentation process, thus avoiding formation of a pomace cap.

The design of the cooling device is derived from the design of the degassing device in that the inner tubular spaces of all passably connected tubular arms and links can be used for circulation of coolant through the degassing device, which, with some additional structural arrangements, makes it a cooling device. The coolant enters on one side of the shaft, proceeds through the internal tubular space of all passably connected tubular arms and links and outlets on the other side of the shaft. Thus formed cooling device is used to control the must temperature during fermentation. Another design feature is that there are two types of couplers connected to the blind holes on both shaft ends. Insert couplers with seals are also provided with connection threads, to which coolant pipes are attached. In the middle part of the insert couplers are non-circular shoulders and slid onto these shoulders are plates with shaped openings, which plates are fastened to the vinificator vessel by bolts. The plates thus prevent the insert couplers from rotating when the degassing and cooling device is rotating and allow a fixed coolant inlet and outlet pipe to be connected.

A design feature of another embodiment of the primary fermentation tank with a mechanical cooling and/or degassing device is that slid onto both ends of the shaft are sleeve couplers with seals. The sleeve couplers are further provided with connection threads, to which coolant pipes are attached. In the middle part of the sleeve couplers are non-circular shoulders. Slid onto the shoulders of the sleeve couplers are plates with shaped openings, which plates are fastened to the vinificator vessel by bolts.

### Brief description of the drawings

The invention will be further illustrated in the drawings, where Fig. 1 shows a side sectional view of the primary fermentation tank with a mechanical cooling and/or degassing device. Fig. 2 shows an axonometric view of the degassing device. Fig. 3 shows a cross-section of a shaft end according to one embodiment. Fig. 4 shows a cross-section of a shaft end according to the other embodiment.

It is understood that the individual embodiments of the device of the present invention are shown by way of illustration only and not as limitations. Those skilled in the art will recognize, or be able to ascertain using no more than routine experimentation, many equivalents to the specific embodiments of the design according to the present invention, which will be specifically described herein. Such equivalents are intended to be encompassed by the following claims. Those skilled in the art would have no problem dimensioning such device and choosing suitable materials and design configurations, which is why these features were not designed in detail.

### Description of the preferred embodiments

### Example 1

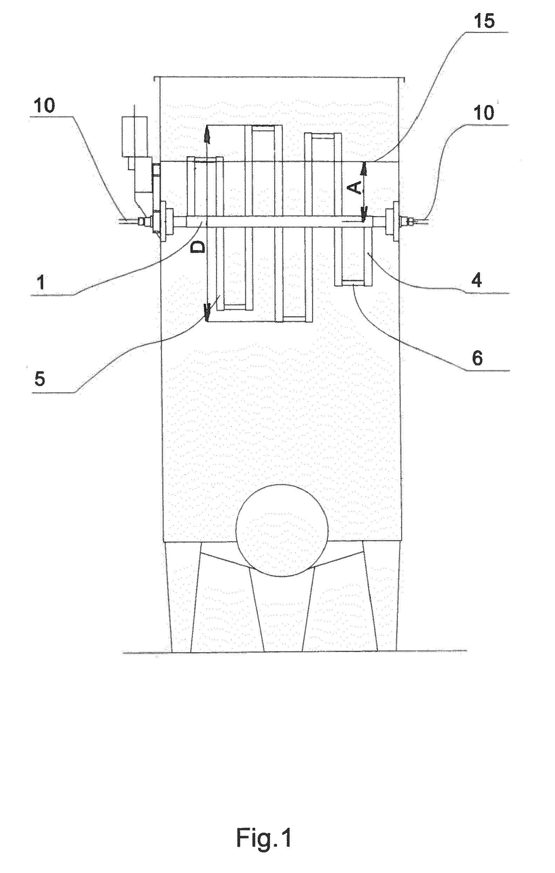

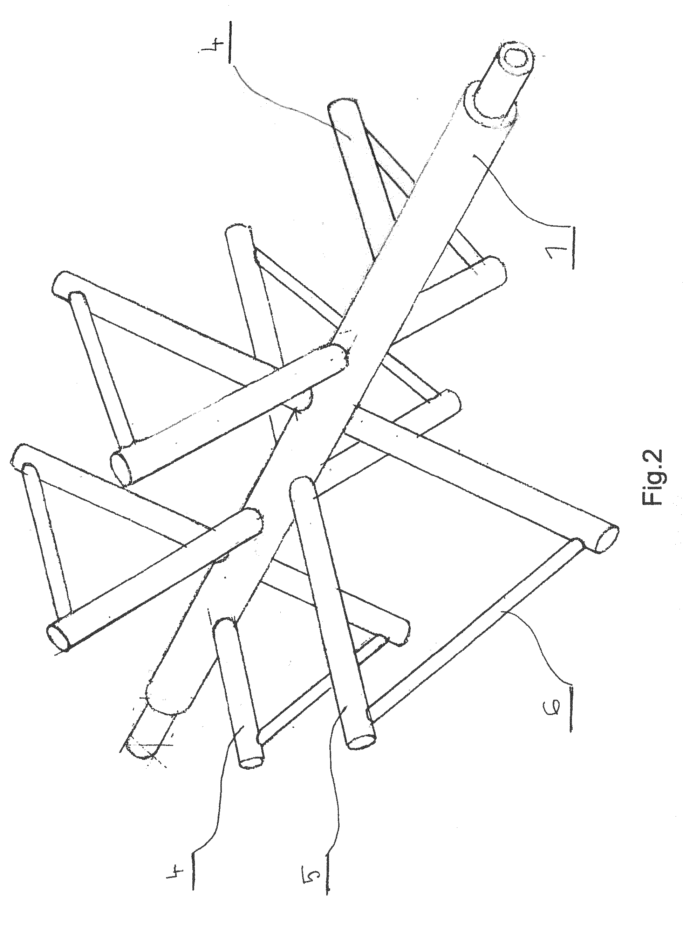

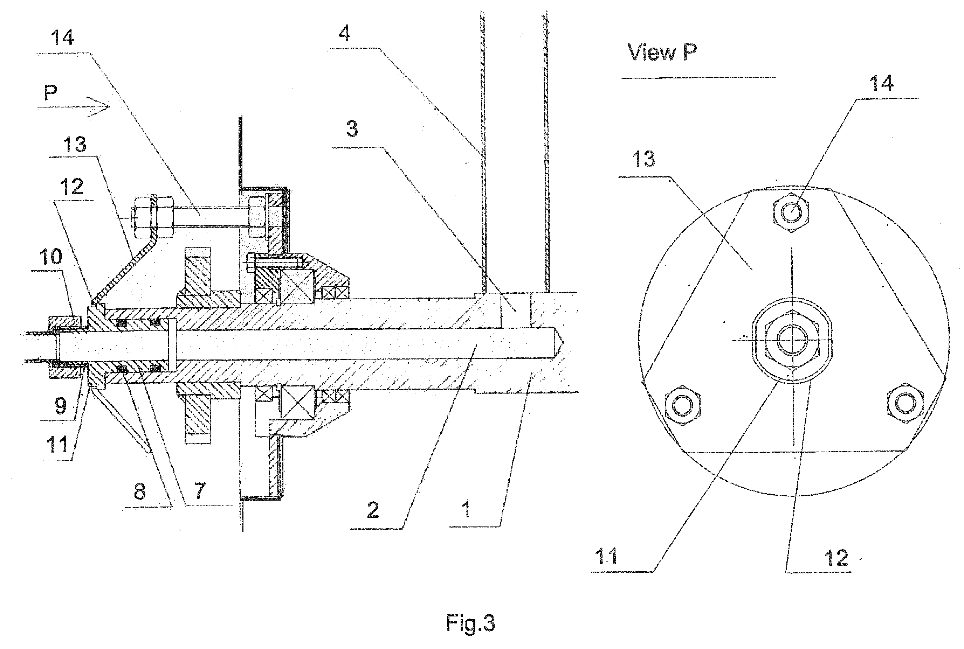

This example describes the design of a primary fermentation tank with a mechanical cooling and/or degassing device according to the present invention for processing of must made from blue grape varieties, which device is shown in Figs. 1, 2 and 3. The actual primary fermentation tank comprises a vertical cylindrical vessel with an open top, conical bottom, three-legged support and a discharge opening with a door at the lowest point of the tank. Attached to the top of the tank is a geared electric motor that drives, via a chain transmission, the degassing and cooling device rotary housed in bearings. The rotary degassing and cooling device comprises a shaft 1 at both ends of which are blind axial holes 2 with radial outlets 3 and welded to these outlets at the shaft 1 are outer tubular arms 4 closed at their ends. In the middle part of the shaft 1, extending transversely through it, are inner tubular arms 5, which are also closed at the ends. Adjacent inner tubular arms 4, 5 are always angularly offset from one another and interconnected by interconnecting tubular links 6, thus creating a three-dimensional tubular structure 17. Inserted into the blind holes 2 in the shaft 1 are insert couplers 7 with seals 8. The insert couplers 7 are also provided with connection threads 9 to which coolant pipes 10 are attached. In the middle part of the insert couplers 7 are non-circular shoulders 11. Slid onto the shoulders 11 of insert couplers 7 are plates 13 having openings 12 shaped to fit the shoulder 11 of the insert 7, which plates are fastened to the vinificator vessel by bolts 14. The shaft 1 is mounted horizontally below the must surface 15 at a distance A less than half of the maximum diameter D of the three-dimensional tubular structure 17.

### Example 2

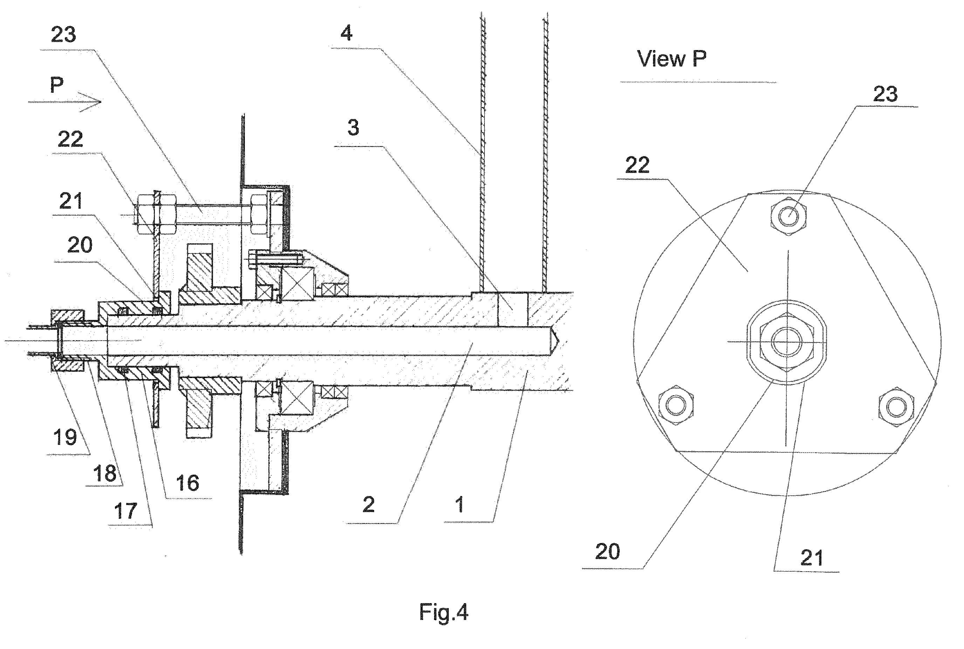

This example describes another design of a primary fermentation tank with a mechanical cooling and/or degassing device according to the present invention for processing of must made from blue grape varieties. The design has been sufficiently described in Example 1, with the only difference being the method of connection of the coolant pipe as shown in Fig. 4. Slid onto both ends of the shaft 1 are sleeve couplers 16 with seals 8. The sleeve couplers 16 are also provided with connection threads 9, to which coolant pipes 10 are attached. In the middle part of the sleeve couplers 16 are shoulders 11 of the sleeve couplers 16. Slid onto them are plates 13 with shaped openings 12, which plates are fastened to the vinificator vessel by bolts 14.

### Example 3

This example describes yet another design of a primary fermentation tank with a mechanical cooling device according to the present invention for cryomaceration of must made from white grape varieties. The design has been sufficiently described in Example 1. The three-dimensional tubular structure 17 rotates, but not for the purpose of degassing. It stirs, but mainly cools the must.

### Industrial applicability

The primary fermentation tank with mechanical cooling and/or degassing device according to the present invention has its use in the wine making industry for primary fermentation of must of blue grape varieties and also for cryomaceration of must of white grape varieties. The primary fermentation tank with a mechanical cooling and/or degassing device may also be used for processing of other types of fruits.

## Claims

1. A primary fermentation tank with a mechanical cooling and/or degassing device comprising a vessel fitted with a bottom discharge opening, **characterized in that** attached to the vessel walls, housed in bearings, is a driven horizontal shaft (1) at both ends of which are blind axial holes 2 with radial outlets 3 and fixedly attached to these outlets (3) on the shaft (1) are outer tubular arms (4); in its middle part, the shaft (1) is provided with spaced radial through holes angularly offset from one another, connected to which are inner tubular arms (5), wherein the adjacent ends of the inner tubular arms (5) are continually interconnected by interconnecting tubular links (6) to form a three-dimensional tubular structure (17), where connected to the outlets of the end interconnecting tubular links (6) are the end parts of the outer tubular arms (4), wherein the blind holes (2) at both ends of the shaft (1) are fitted with insert or sleeve couplers (7, 16) for inlet and outlet of coolant.

2. A primary fermentation tank with a mechanical cooling and/or degassing device according to claim 1, **characterized in that** inserted into the blind holes (2) of the shaft (1) are insert couplers (7) with seals (8), which are further provided with connection threads (9), fastened to which are coolant pipes (10); in the middle part of the insert couplers (7) are shaped shoulders (11) and slid onto them are plates (13) with shaped openings (12), which plates are fastened to the vessel by bolts (14).

3. A primary fermentation tank with a mechanical cooling and/or degassing device according to claim 1, **characterized in that** slid onto the ends of the shaft (1) are sleeve couplers (16) with seals (8) and fastened to these sleeve couplers (16), by means of the connection threads (9), are coolant pipes (10); in the middle part of the sleeve couplers (16) are shaped shoulders (11) and slid onto them are plates (13) with shaped openings (12), which plates are fastened to the vessel by bolts (14).

4. A primary fermentation tank with a mechanical cooling and/or degassing device according to claim 1, **characterized in that** the shaft (1) is mounted below the must surface level (15) at a distance (A) less than half of the maximum diameter (D) of the three-dimensional tubular structure (17).
